Europäisches Patentamt

European Patent Office

Office européen des brevets

(19)

(11) Veröffentlichungsnummer : **0 340 416 B1**

## (12) EUROPÄISCHE PATENTSCHRIFT

(45) Veröffentlichungstag der Patentschrift :
13.05.92 Patentblatt 92/20

(51) Int. Cl.⁵ : **C07C 21/06,** C07C 17/08

(21) Anmeldenummer : 89104051.1

(22) Anmeldetag : 08.03.89

(54) **Verfahren zur Herstellung von Vinylchlorid durch Umsetzung von Acetylen mit Chlorwasserstoff.**

(30) Priorität : 30.04.88 DE 3814785
20.07.88 DE 3824634

(43) Veröffentlichungstag der Anmeldung :
08.11.89 Patentblatt 89/45

(45) Bekanntmachung des Hinweises auf die
Patenterteilung :
13.05.92 Patentblatt 92/20

(84) Benannte Vertragsstaaten :
AT BE CH DE ES FR GB IT LI NL SE

(56) Entgegenhaltungen :
DE-A- 3 009 520
FR-A- 1 431 352
PATENT ABSTRACTS OF JAPAN, unexamined
applications, Sektion C, Band 2, Nr. 34, 8. MUrz
1978 THE PATENT OFFICE JAPANESEGO-
VERNMENT Seite 4499 C 77

(73) Patentinhaber : HÜLS
AKTIENGESELLSCHAFT
- RSP Patente / PB 15 - Postfach 13 20
W-4370 Marl 1 (DE)

(72) Erfinder : Thelen, Gerhard, Dr.
Sepp-Herberger-Strasse 62
W-4405 Nottuln (DE)
Erfinder : Bartels, Harald, Dr.
Käthe-Kollwitz-Strasse 37
W-4370 Marl (DE)
Erfinder : Droste, Wilhelm, Dr.
Pommernstrasse 4 a
W-4370 Marl (DE)
Erfinder : Deppe, Herbert
Bullenkempe 13
W-4280 Borken (DE)

## Beschreibung

Die Erfindung betrifft ein Verfahren zur Herstellung von Vinylchlorid durch Umsetzung von Acetylen mit Chlorwasserstoff in Gegenwart eines Edelmetall-Katalysators in einem organischen Lösungsmittel bei Temperaturen höher als Raumtemperatur.

Die großtechnische Herstellung von Vinylchlorid durch Umsetzung von Acetylen mit Chlorwasserstoff wird üblicherweise in der Gasphase an einem heterogenen $HgCl_2$-haltigen Kontakt durchgeführt. Aus ökologischen Gründen besteht großes Interesse, einen quecksilberchloridfreien Katalysator einzusetzen. Alternative Vorschläge für ein Gasphasenverfahren konnten sich nicht durchsetzen.

Für die Durchführung der Hydrochlorierung von Acetylen in der flüssigen Phase gibt es in der Literatur ebenfalls einige Beispiele, die aber großtechnisch nicht praktikabel sind:

So ist bekannt, daß man gemäß US-PS 1 812 542, US-PS 1 934 324 und US-PS 3 113 158 Acetylen zu Vinylchlorid mit einer wäßrigen Salzsäure umsetzen kann, in der als Katalysator eine Kupferverbindung, vorzugsweise Kupferchlorid, gelöst ist. Gemäß US-PS 1 812 542 enthält die Katalysatorlösung noch zusätzlich Ammoniumchlorid, gemäß US-PS 1 934 324 ein Alkalimetall- oder Erdalkalimetallchlorid und gemäß US-PS 3 113 158 ein Phosphin und/oder Diphosphin und/oder Methylphosphin.

SU-PS 165 446 beschreibt die Hydrochlorierung von Acetylen an Kupferchlorid in Dimethylformamid als Lösungsmittel und SU-PS 232 956 an Kupferchlorid in salzsaurer Triethanolaminlösung.

Die Angaben zum Acetylenumsatz reichen von 26 bis 99 %. Es werden Raum-Zeit-Ausbeuten

$$\left(\frac{Produkt}{Katalysatorvolumen \cdot Zeit} \left[\frac{Gramm}{Liter \cdot Stunde}\right]\right)$$

von 10 bis 130 g Vinylchlorid/l · h angegeben. Von Nachteil ist in diesen Fällen die hohe Konzentration von bis zu 60 Gewichtsprozent an Kupferchlorid in den verwendeten Lösungsmitteln. Unbefriedigend ist außerdem, daß das Katalysatorsystem, wie oben beschrieben, zumeist noch zusätzliche Bestandteile enthält.

JP-OS 77/136 103 offenbart ein Verfahren zur Herstellung von Vinylchlorid aus Acetylen und Chlorwasserstoff in Gegenwart von Goldchlorid und Platinchlorid und/oder Palladiumchlorid als Katalysator. Als Lösungs- bzw. Suspensionsmittel werden Wasser oder organische Lösungsmittel, wie z. B. Paraffine, Chlorkohlenwasserstoffe, Diisopropylbenzol, Chloralhydrat und Ethylenchlorhydrin verwendet. Die katalytisch aktiven Metallchloride können auch mit Aktivkohle als Trägermaterial suspendiert werden. Gemäß Anspruch 2 wird darüber hinaus noch ein Übergangsmetallchlorid als zusätzliche Katalysatorkomponente eingesetzt. Es werden Umsätze von 54 bis 74 % bei Selektivitäten um 99 % erzielt. Die Raum-Zeit-Ausbeute beträgt etwa 100 g Vinylchlorid/l · h. Nachteilig ist, daß mindestens zwei verschiedene Edelmetallchloride anwesend sein müssen und nur geringe Umsatzraten erreicht werden.

Der Erfindung liegt die Aufgabe zugrunde, Vinylchlorid durch Hydrochlorierung von Acetylen in hoher Raum-Zeit-Ausbeute bei gleichzeitig hohem Umsatz und hoher Selektivität in homogener Katalysatorlösung mit geringer Katalysatorkonzentration auf einfache Weise herzustellen.

Überraschenderweise konnten bei Einsatz einer Palladiumverbindung als Katalysator in einem Carbonsäureamid als Lösungsmittel Raum-Zeit-Ausbeuten erzielt werden, die um eine Zehnerpotenz größer als die gemäß dem Stand der Technik sind, und zwar bei nahezu vollständigen Acetylenumsätzen und Selektivitäten bis hinauf zu 99 %.

Gegenstand der vorliegenden Erfindung ist daher ein Verfahren zur Herstellung von Vinylchlorid durch Umsetzung von Acetylen mit Chlorwasserstoff in Gegenwart eines Edelmetall-Katalysators in einem organischen Lösungsmittel bei Temperaturen, die übe dem Schmelzpunkt des Hydrochlorids des als Lösungsmittel verwendeten Carbonsäureamids liegen, dadurch gekennzeichnet, daß man als Katalysator eine Palladiumverbindung in einem aliphatischen und/oder cycloaliphatischen Carbonsäureamid als Lösungmittel einsetzt.

Als Katalysatoren geeignet sind Palladiumverbindungen, wie beispielsweise Palladium(II)chlorid, Alkali- und Erdalkalitetrachloropalladate - z. B. $Na_2(PdCl_4)$, $K_2(PdCl_4)$, $Li_2(PdCl_4)$ und $(NH_4)_2(PdCl_4)$ - Hydrogentetrachloropalladat(II), Palladium(II)acetat, Palladiumacetylacetonat, Bis(triphenylphosphin)palladium(II)chlorid, Bis(acetonitril)palladium(II)chlorid und Bis(benzonitril)palladium(II)chlorid. Bevorzugt wird Palladium(II)chlorid als Katalysator eingesetzt.

Man führt die Umsetzung von Acetylen mit Chlorwasserstoff in der Flüssigphase in einem aliphatischen Carbonsäureamid als Lösungsmittel durch. Das Carbonsäureamid kann cyclischer oder acyclischer Struktur sein. Beispielsweise handelt es sich um

a) Carbonsäureamide der allgemeinen Formel $R_1CONR_2R_3$, wobei

R$_1$ und R$_2$ für Wasserstoff oder für einen Alkylrest mit 1 bis 5 C-Atomen stehen und

R$_3$ für einen Alkylrest mit 1 bis 5 C-Atomen steht, wie z. B. Ameisensäuredimethylamid, Essigsäuredimethylamid, und um

b) Carbonsäureamide der allgemeinen Formel R$_1$CONR$_2$R$_3$, wobei

R$_3$ für Wasserstoff oder für einen Alkylrest mit 1 bis 5 C-Atomen steht und

R$_1$, R$_2$ einen Ringschluß bilden mit 2 bis 5 C-Atomen, insbesondere mit 3 bis 4 C-Atomen. N-Methylpyrrolidon wird bevorzugt als Lösungsmittel verwendet.

Um den erfindungsgemäß eingesetzten Katalysator in dem erfindungsgemäß verwendeten Lösungsmittel aufzulösen, empfiehlt es sich, das Lösungsmittel vorher, eventuell unter Erwärmen, mit Chlorwasserstoff, z. B. durch Einleiten, anzureichern. Eine Sättigung des Lösungsmittels mit Chlorwasserstoff ist in der Regel nicht erforderlich.

Das erfindungsgemäße Verfahren wird durchgeführt, indem man die Komponenten Acetylen und Chlorwasserstoff bevorzugt in einem Molverhältnis von 1 : 1 bis 1 : 3, besonders bevorzugt von 1 : 1 bis 1 : 1,5, in Gegenwart des Katalysators zur Reaktion bringt.

Bevorzugt setzt man, bezogen auf die verwendete Menge Lösungsmittel, 0,1 bis 1,0 Gewichtsprozent Katalysator, besonders bevorzugt 0,3 bis 0,8 Gewichtsprozent, ein.

Acetylen und Chlorwasserstoff werden bei Temperaturen höher als Raumtemperatur umgesetzt, in jedem Fall muß die Temperatur über dem Schmelzpunkt des Hydrochlorids des erfindungsgemäß verwendeten Carbonsäureamids liegen.

Bevorzugt führt man die Umsetzung bei einer Temperatur von 100 °C bis 200 °C, besonders bevorzugt von 140 °C bis 170 °C, durch.

Die beiden Edukte Acetylen und Chlorwasserstoff werden synchron, vorzugsweise vorgemischt, in die Katalysatorlösung eingeleitet, d. h. das Reaktionssystem ist eine Blasensäule.

Die Umsetzung findet dabei unter Atmosphärendruck oder unter leicht erhöhtem Druck statt.

Die Erfindung wird durch die folgenden Beispiele näher erläutert:

Beispiel 1

Die Umsetzung von Acetylen mit Chlorwasserstoff wird folgendermaßen durchgeführt:

Ein senkrechtstehendes, doppelwandiges Glasrohr mit einem Innendurchmesser des Innenrohres von 2,2 cm wird mit einer Lösung von 175 mg Na$_2$PdCl$_4$ in 45 ml N-Methylpyrrolidon gefüllt. Chlorwasserstoff mit einem Volumenstrom von 7,2 Nl/h (Normliter/Stunde) und Acetylen mit einem Volumenstrom von 5,9 Nl/h werden vor dem Reaktor vereinigt und gemischt und der auf 150 °C temperierten Katalysatorlösung von unten zugeführt (Wärmeträger im Außenmantel). Das aus dem Reaktor strömende, gasförmige Produktgemisch wird gaschromatographisch analysiert und ein Umsatz des Acetylens von 99,7 % bei einer Selektivität zu Vinylchlorid von 98,3 % ermittelt. Die Raum-Zeit-Ausbeute beträgt 358 g Vinylchlorid/l · h.

Beispiel 2

Führt man die Reaktion analog zu Beispiel 1 in Gegenwart von 150 mg PdCl$_2$, gelöst in 38 ml N.N-Dimethylformamid, bei einer Temperatur von 120 °C durch, so erhält man bei Einsatz von 11,25 Nl/h Acetylen und 11,25 Nl/h Chlorwasserstoff einen Acetylenumsatz von 80 % und eine Selektivität von 98,7 %.

Die Raum-Zeit-Ausbeute beträgt 652 g Vinylchlorid/l · h.

Beispiel 3

Analog zu Beispiel 2 erzielt man bei Einsatz von 5,9 Nl/h Acetylen und 7,2 Nl/h Chlorwasserstoff einen Umsatz von 94,7 % und eine Selektivität von 98,9 %.

Die Raum-Zeit-Ausbeute beträgt 405 g Vinylchlorid/l · h.

Beispiel 4

Analog zu Beispiel 1 werden in Gegenwart von 21 ml N-Methylpyrrolidon-Lösung, die 4,7 g PdCl$_2$ pro l enthält, bei Einsatz eines Gasgemisches aus 13 Nl/h Acetylen und 14,3 Nl/h Chlorwasserstoff 96,0 % Acetylenumsatz und eine Selektivität zu Vinylchlorid von 98,8 % erzielt. Die Raum-Zeit-Ausbeute beträgt 1 638 g Vinylchlorid/l · h.

Beispiel 5

Führt man die Reaktion wie in Beispiel 4 beschrieben, allerdings in Anwesenheit von 3 g PdCl$_2$/l, mit 12,5 Nl/h Acetylen und 15 Nl/h Chlorwasserstoff durch, so beträgt der Acetylenumsatz 89,5 % und die Selektivität zu Vinylchlorid 98,7 %.
Die Raum-Zeit-Ausbeute beträgt 1467 g Vinylchlorid/l · h.

Beispiel 6

Analog zu Beispiel 1 wird in Gegenwart von 30 ml N-Methylpyrrolidon-Lösung, die 4 g PdCl$_2$/l enthält, bei einer Temperatur von 148 °C und einem Edukteinsatz von 13 Nl/h Acetylen und 15,2 Nl/h Chlorwasserstoff ein Acetylenumsatz von 95,9 % erzielt.
Die Selektivität zu Vinylchlorid beträgt 99,2 % und die Raum-Zeit-Ausbeute 1 150 g Vinylchlorid/l · h.

Beispiel 7

Führt man die Reaktion analog zu Beispiel 6 in Gegenwart von 4,9 g PdCl$_2$/l durch, so beträgt bei einer Temperatur von 160 °C unter Einsatz von 12 Nl/h Acetylen und 14,4 Nl/h Chlorwasserstoff der Acetylenumsatz 99,6 %, die Selektivität 99,3 % und die Raum-Zeit-Ausbeute 1 104 g Vinylchlorid/l · h.

## Patentansprüche

1. Verfahren zur Herstellung von Vinylchlorid durch Umsetzung von Acetylen mit Chlorwasserstoff in Gegenwart eines Edelmetall-Katalysators in einem organischen Lösungsmittel bei Temperaturen, die über dem Schmelzpunkt des Hydrochlorids des als Lösungsmittel verwendeten Carbonsäureamids liegen, dadurch gekennzeichnet, daß man als Katalysator eine Palladiumverbindung in einem aliphatischen und/oder cycloaliphatischen Carbonsäureamid als Lösungsmittel einsetzt.

2. Verfahren nach Anspruch 1, dadurch gekennzeichnet, daß man als Katalysator Palladium(II)chlorid einsetzt.

3. Verfahren nach den Ansprüchen 1 und 2, dadurch gekennzeichnet, daß man als Lösungsmittel N-Methylpyrrolidon verwendet.

4. Verfahren nach den Ansprüchen 1 bis 3, dadurch gekennzeichnet, daß man, bezogen auf die verwendete Menge Lösungsmittel, 0,1 bis 1,0 Gewichtsprozent Katalysator einsetzt.

5. Verfahren nach Anspruch 4, dadurch gekennzeichnet, daß man, bezogen auf die verwendete Menge Lösungsmittel, 0,3 bis 0,8 Gewichtsprozent Katalysator einsetzt.

6. Verfahren nach den Ansprüchen 1 bis 5, dadurch gekennzeichnet, daß man die Umsetzung bei einer Temperatur von 100 bis 200 °C durchführt.

7. Verfahren nach Anspruch 6, dadurch gekennzeichnet, daß man die Umsetzung bei einer Temperatur von 140 bis 170 °C durchführt.

8. Verfahren nach den Ansprüchen 1 bis 7, dadurch gekennzeichnet, daß man Acetylen und Chlorwasserstoff in einem Molverhältnis von 1 : 1 bis 1 : 3 zur Reaktion bringt.

9. Verfahren nach Anspruch 8, dadurch gekennzeichnet, daß man Acetylen und Chlorwasserstoff in einem Molverhältnis von 1 : 1 bis 1 : 1,5 zur Reaktion bringt.

## Claims

1. A process for the preparation of vinyl chloride by reacting acetylene with hydrogen chloride in the presence of a precious metal catalyst in an organic solvent at temperatures above the melting point of the hydrochloride of the carboxylic acid amide used as solvent, characterized in that the catalyst used is a palladium compound in an aliphatic and/or cycloaliphatic carboxylic acid amide as solvent.

2. A process according to claim 1, characterized in that the catalyst used is palladium (II) chloride.

3. A process according to claims 1 and 2, characterized in that the solvent used is N-methyl pyrrolidone.

4. A process according to claims 1 to 3, characterized in that 0.1 to 1.0% by weight of catalyst is used, referred to the quantity of solvent used.

5. A process according to claim 4, characterized in that 0.3 to 0.8% by weight of catalyst is used, referred to the quantity of solvent used.

6. A process according to claims 1 to 5, characterized in that the reaction is performed at a temperature of 100 to 200°C.

7. A process according to claim 6, characterized in that the reaction is performed at a temperature of 140 to 170°C.

8. A process according to claims 1 to 7, characterized in that acetylene and hydrogen chloride are reacted in a molar ratio of 1 : 1 to 1 : 3.

9. A process according to claim 8, characterized in that acetylene and hydrogen chloride are reacted in a molar ratio of 1 : 1 to 1 : 1.5.

**Revendications**

1. Procédé de préparation de Chlorure de Vinyle par réaction de l'acétylène avec l'acide Chlorhydrique en présence d'un catalyseur en métal noble dans un solvant organique à des températures qui se situent au dessus du point de fusion du chlorhydrate du Carboxamide acide utilisé comme solvant, caractérisé en ce que l'on met en oeuvre comme catalyseur un composé de palladium dans un carboxamide acide aliphatique et/ou cycloaliphatique comme solvant.

2. Procédé selon la revendication 1, caractérisé en ce que l'on met en jeu comme catalyseur du chlorure de Palladium (II).

3. Procédé selon les revendications 1 et 2 caractérisé en ce que l'on utilise comme solvant la N-méthylpyrrolidone.

4. Procédé selon les revendications 1 à 3, caractérisé en ce que l'on met en jeu 0,1 à 1,0 % en poids de catalyseur rapporté à la quantité utilisée de solvant.

5. Procédé selon la revendication 4, caractérisé en ce que l'on met en jeu 0,3 à 0,8 % en poids de catalyseur rapporté à la quantité de solvant utilisée.

6. Procédé selon les revendications 1 à 5, caractérisé en ce que l'on effectue la réaction à une température allant de 100 à 200° C.

7. Procédé selon la revendication 6, caractérisé en ce que l'on effectue la réaction à une température allant de 140 à 170° C.

8. Procédé selon les revendications 1 à 7, caractérisé en ce que l'on met en réaction l'acétylène et l'acide chlorhydrique dans un rapport molaire de 1 ; 1 à 1 ; 3.

9. Procédé selon la revendication 8, caractérisé en ce que l'on met en réaction l'acétylène et l'acide chlorhydrique dans un rapport molaire allant de 1 : 1 à 1 ; 1,5.